# EUROPÄISCHE PATENTSCHRIFT

(11) **EP 2 276 562 B1**
(45) Veröffentlichungstag und Bekanntmachung des Hinweises auf die Patenterteilung: **26.06.2019**
(21) Anmeldenummer: 09723248.2
(22) Anmeldetag: 16.03.2009
(51) Int. Cl.: B01J 23/72, B01J 23/50, C07C 45/00, C07C 45/38, C07C 45/39, C07C 47/21, B01J 23/38

(54) **VERWENDUNG EINES GETRÄGERTEN EDELMETALLHALTIGEN KATALYSATORS ZUR OXIDATIVEN DEHYDRIERUNG**
USE OF A SUPPORTED CATALYST CONTAINING PRECIOUS METAL FOR OXIDATIVE DEHYDROGENATION
UTILISATION D'UN CATALYSEUR SUPPORTÉ CONTENANT DES MÉTAUX PRÉCIEUX POUR LA DÉSHYDROGÉNATION OXYDATIVE

(30) Priorität: 19.03.2008 DE 102008014910
(43) Veröffentlichungstag der Anmeldung: 26.01.2011
(73) Patentinhaber: BASF SE, 67056 Ludwigshafen am Rhein (DE)
(72) Erfinder: MÄURER, Torsten, 67245 Lambsheim (DE); SEEBER, Georg, 67245 Lambsheim (DE); ABDALLAH, Radwan, 67063 Ludwigshafen (DE); JOHANN, Thorsten, 67117 Limburgerhof (DE); WEGNER, Günter, 67354 Römerberg (DE)
(86) Internationale Anmeldenummer: PCT/EP2009/053081
(87) Internationale Veröffentlichungsnummer: WO 2009/115492

(56) Entgegenhaltungen:
- EP-A- 0 357 293
- EP-A- 0 415 745
- EP-A- 0 881 206
- WO-A-2008/037693

## Beschreibung

Die vorliegende Erfindung betrifft die Verwendung eines geträgerten silberhaltigen Katalysators zur Herstellung von olefinisch ungesättigten Carbonylverbindungen aus olefinisch ungesättigten Alkoholen durch oxidative Dehydrierung sowie entsprechende geträgerte silberhaltige Katalsysatoren. Insbesondere betrifft die vorliegende Erfindung die Verwendung von nach einem bestimmten Verfahren erhältlichen geträgerten Silberkatalysator zur Herstellung von 3-Methylbut-2-en-1-al aus 3-Methylbut-3-en-1-ol.

Die Herstellung von alpha,beta-ungesättigten Carbonylverbindungen durch oxidative Dehydrierung an geeigneten Katalysatoren ist dem Fachmann bekannt und vielfach in der Literatur beschrieben.

In der DE-B-20 20 865 wird dementsprechend ein Verfahren zur Herstellung von alpha,beta-ungesättigten Carbonylverbindungen beschrieben, wobei als Dehydrierungskatalysatoren ausweislich der Beschreibung Legierungen und Metallverbindungen, speziell einige Metalloxide der Nebengruppenelemente eingesetzt werden können. Weiterhin wird in dieser Schrift ausgeführt, dass die Katalysatoren in reiner Form wie in Form von Mischkatalysatoren mit oder ohne Trägersubstanz verwendet werden können. Als besonders geeignet werden Zinkoxid, Kadmiumoxid und Manganoxid sowie Mischkatalysatoren aus den Metallen Cu, Ag und/oder Zn genannt. Zur Herstellung des Katalysators finden sich in dieser Schrift keine weiteren Angaben.

In der EP-A 881 206 wird ein Verfahren zur kontinuierlichen technischen Herstellung ungesättigter aliphatischer Aldehyde in einem Rohrbündelreaktor beschrieben. Als bevorzugte Katalysatoren für dieses Verfahren werden Silber-Trägerkatalysatoren genannt, die aus Kugeln eines inerten Trägermaterials bestehen, die mit 0,1 bis 20 Gew.-%, bezogen auf die Menge des Trägers, einer Schicht aus metallischem Silber in Form einer glatten, abriebfesten Schale beschichtet sind. Weiterhin soll ein bestimmtes Verhältnis des größten Durchmessers der beschichteten Katalysatorkugeln zum Innendurchmesser des Reaktionsrohrs vorzugsweise eingehalten werden.

Aus der DE-A 27 15 209 ist ein Verfahren zur Herstellung von 3-Alkylbuten-1-alen bekannt, wobei ein Katalysator mit einer Gesamtschichtdicke von 5 bis 35 mm und 2 oder mehr Schichten Silber- und/oder Kupferkristallen eingesetzt wird. Die Herstellung des Katalysators mit mehreren Schichten des Edelmetalls ist dabei relativ aufwändig.

Aus der EP-A 357 292 ist ein Verfahren zur Herstellung von Ethylenoxid bekannt. Als Katalysatoren in diesem Verfahren werden Silberkatalysatoren eingesetzt, wobei das Silber auf einem porösen hitzebeständigen Träger mit einer bestimmten spezifischen Oberfläche nach BET aufgebracht ist. Ausweislich den Angaben in dieser Schrift kann das Silber auf den Träger als Suspension von Silber oder Silberoxid in einem flüssigen Medium, beispielsweise Wasser, oder durch Tränkung des Trägers mit einer Lösung einer Silberverbindung aufgebracht werden. Anschließend wird diese Silberverbindung zu elementarem Silber durch thermische Behandlung reduziert. Auf eine mögliche Verwendung der so hergestellten silberhaltigen geträgerten Katalysatoren zur Herstellung von ethylenisch ungesättigten Carbonylverbindungen finden sich in dieser Schrift keine Hinweise.

3-Methylbut-2-en-1-al, auch unter dem Trivialnamen Prenal bekannt, ist eine wichtige Vorstufe für Citral, welches wiederum ein wichtiges Produkt für eine Vielzahl von chemischen Synthesen darstellt. Die in der Literatur beschriebenen Katalysatoren zur Herstellung von Prenal (3-Methylbut-2-en-1-al) werden nach relativ komplexen Verfahren und unter Produktionsbedingungen hergestellt, die insgesamt verbesserungsbedürftig sind. Wünschenswert wäre daher, edelmetallhaltige Trägerkatalysatoren für die Synthese von Prenal aus Isoprenol (3-Methylbut-3-en-1-ol) zu erhalten, die auf einfache Weise zugänglich sind und auch hinsichtlich ihrer Selektivität einfach durch Zusätze von als Promotoren wirkenden Verbindungen gesteuert werden können.

Erfindungsgemäß wird diese Aufgabe durch die Verwendung gemäß Anspruch 1 zur Herstellung von olefinisch ungesättigten Carbonylverbindungen aus olefinisch ungesättigten Alkoholen durch oxidative Dehydrierung gelöst.

Die vorliegende Erfindung betrifft weiterhin ein Verfahren zur Herstellung eines geträgerten silberhaltigen Katalysators zur oxidativen Dehydrierung von olefinisch ungesättigten Alkoholen gemäß Anspruch 4.

Schließlich betrifft die vorliegende Erfindung geträgerte silberhaltige Katalysatoren gemäß Anspruch 6.

Der spezifische Widerstand wird in einer Messzelle ermittelt, deren Boden aus Edelstahl und deren Mantel aus isolierendem Kunststoff besteht (Innendurchmesser 10 mm, Höhe 32 cm, Katalysatorvolumen etwa 25 ml). Der Katalysator wird eingefüllt und etwas gerüttelt, um eine gleichmäßige Katalysatorschüttung zu erreichen. Danach wird ein Edelstahlstempel auf die Katalysatorschüttung aufgesetzt. Stempel und Boden dienen in dieser Messanordnung als Messelektroden. Zur Widerstandsmessung wird ein Strommessgerät mit der Probe in Reihe geschaltet und mit einem Netzgerät eine Spannung zwischen 10 mV und 5 V eingestellt. Der zugehörige Strom wird registriert und der spezifische Widerstand wird berechnet. Die Messung wird bei atmosphärischem Druck und einer Luftfeuchtigkeit von max. 50% bei einer Temperatur im Bereich von 22 - 25°C durchgeführt.

Bevorzugte Ausführungsformen der Erfindung sind den Unteransprüchen und der nachfolgenden Beschreibung und den Beispielen zu entnehmen.

Bei der erfindungsgemäßen Verwendung wird ein geträgerter silberhaltiger Katalysator eingesetzt, welcher dadurch erhältlich ist, dass eine komplexierte schwerlösliche Silberverbindung, ggf. in Mischung mit als Promotoren wirkenden Zusätzen, aus Suspension oder Lösung auf Steatit als Trägermaterial aufgebracht wird.

Die Silberverbindung liegt in den Suspensionen oder Lösungen, aus denen sie komplexiert auf Steatit als Trägermaterial aufgebracht wird, vorzugsweise in Anteilen, berechnet als Edelmetall, im Bereich von 0,5 bis 50 Gew.-%, vorzugsweise im Bereich von 1 bis 40 Gew.-% und besonders bevorzugt im Bereich von 5 bis 35 Gew.-% vor.

Prinzipiell eignen sich alle Silberverbindungen die in wässriger Lösung bei einer Temperatur von 25°C und einem pH-Wert von 7 eine Löslichkeit von weniger als 5,0 g/l, besonders bevorzugt von weniger als 1 g/l und besonders bevorzugt von weniger als 0,5 g/l haben. Nur beispielhaft seien hier als Verbindungen des Silbers Silberoxalat, Salze des Silbers mit gesättigten oder ungesättigten Mono-Carbonsäuren, wie z. B. Ameisensäure, Essigsäure, Propionsäure, Buttersäure, Milchsäure oder auch Salze der Benzoesäure oder Salicylsäure genannt. Weiterhin eignen sich Verbindungen von gesättigten oder ungesättigten Di-Carbonsäuren wie Fumarsäure oder Maleinsäure oder von gesättigten oder ungesättigten Tri-Carbonsäuren wie z. B. Zitronensäure oder deren Salzen. Grundsätzlich eignen sich, wie vorstehend erwähnt, alle schwer löslichen Verbindungen der Edelmetalle, so dass die vorstehende Aufzählung nur als beispielhafte Aufzählung zu verstehen ist.

Bei der erfindungsgemäßen Verwendung werden die schwer löslichen Silberverbindungen als Feststoff mit Komplexbildnern oder Lösungen von Komplexbildnern in Kontakt gebracht. Erfindungsgemässe Komplexbildner sind amin-, hydroxy- und oder Thio-Makrozyklen.

Nach einer besonders bevorzugten Verwendung werden als schwerlösliche Salze Oxalate, insbesondere Silberoxalat eingesetzt.

Die schwerlöslichen Silberverbindungen werden als Feststoff mit dem Komplexbildner bzw. einer Lösung des Komplexbildners in Kontakt gebracht, wodurch eine komplexierte Lösung oder Suspension der schwerlöslichen Silberverbindung entsteht.

Dieser Suspension oder Lösung der schwerlöslichen Silberverbindungen können als Promotoren geeignete weitere Zusätze zugesetzt werden. Nur beispielhaft seien hier Alkali-, Erdalkali- und Übergangsmetalle (wie Li, Rb, Cs, Ca, Mg, V, Co, Ni, Ir oder Re) erwähnt, die beispielsweise als Halogenide (Fluoride, Chloride) Carboxylate oder Nitrate oder auch in Form schwefelhaltiger Anionen wie Sulfate, Sulfite oder Sulfide eingesetzt werden können. Ebenfalls geeignet sind Phosphate, Cyanide und Hydroxide sowie Carbonate oder deren Mischungen. Schließlich können auch Anionen von Heteropolysäuren, insbesondere von Heteropolysäuren der Elemente der sechsten und siebten Nebengruppe des Periodensystems (Notation gemäß IUPAC-Vorschlag von 1985) eingesetzt werden.

Nach der erfindungsgemäßen Verwendung wird die komplexierte Silberverbindung aus Suspension oder Lösung, welche ggf. wie vorstehend ausgeführt Promotoren enthalten kann, auf Steatit als geeignetes Trägermaterial aufgebracht.

In einer bevorzugten Ausgestaltung der vorliegenden Erfindung sind die Trägermaterialien möglichst wenig porös und weisen eine BET-Oberfläche von maximal 0,1 m²/g auf.

In einer bevorzugten Ausgestaltung der vorliegenden Erfindung sind die Trägermaterialien kugelförmig und weisen einen mittleren Durchmesser von 1,3 bis 2,5 mm auf.

Das erfindungsgemässe Trägermaterial ist Steatit, ein keramischer Werkstoff auf der Basis natürlicher Rohstoffe, der aus der Hauptkomponente Speckstein (Mg(Si₄O₁₀)(OH)₂), einem natürlichem Magnesiumsilikat, besteht. Weiterhin können Zusätze von Ton und Feldspat oder Bariumcarbonat enthalten sein.

Nach dem Aufbringen der schwer löslichen Silberverindung ggf. mit dem Zusatz von Promotoren, aus Suspension oder Lösung auf Steatit als Trägermaterial wird dieses einer thermischen Behandlung bei Temperaturen im Bereich von 100 bis 400°C, vorzugsweise von 120 bis 360°C und besonders bevorzugt von 150 bis 340°C unterworfen. Dies erfolgt für einen Zeitraum im Bereich von 5 Minuten bis 5 Stunden, vorzugsweise 5 Minuten bis 3 Stunden und besonders bevorzugt 10 Minuten bis 1 Stunde.

Durch diese thermische Behandlung wird auf der Oberfläche des Trägermaterials aus der Silberverbindung das Edelmetall selbst gebildet, welches dann die aktive Spezies des geträgerten Katalysators darstellt.

Die Silbergehalte gemessen in Gew.-%, bezogen auf das Trägermaterial, liegen nach der thermischen Behandlung im allgemeinen im Bereich von 0,2 bis 25 Gew.-%, vorzugsweise im Bereich von 0,5 bis 20 Gew.-% und besonders bevorzugt im Bereich von 1 bis 15 Gew.-%.

In einigen Fällen hat es sich als vorteilhaft herausgestellt, bei der Kombination von schwer löslicher Silberverbindung und Komplexbildner eine in situ hergestellte Silberverbindung einzusetzen. Diese Verfahrensvariante sei nachstehend beispielhaft für die Herstellung einer schwer löslichen Silberverbindung beschrieben.

Zur Herstellung der schwerlöslichen Silberverbindung können beliebige Silbersalze beispielsweise Silbernitrat oder andere Silbersalze wie z. B. Silbersulfat, Silberfluorit, Silbertriflat, Silberperchlorat oder auch Tricyanoargentat bzw. Tricyanoargentat-Verbindungen eingesetzt werden.

Weiterhin sind geeignet Silberausgangsstoffe wie Silberchlorid, Silberbromid oder Silberiodid, Silbersulfit oder Silbercarbonat, welche durch geeignete Behandlung mit Säuren wie z. B. Fluorwasserstoff, Salpetersäure oder Schwefelsäure in Lösung gebracht werden können.

Die Lösungen dieser Salze der Silberverbindungen können dann mit Fällungsreagenzien in gelöster Form kombiniert werden, um die schwerlösliche Silberverbindung auszufällen. Dabei kann sowohl das Fällungsreagenz zur Silberlösung gegeben werden, als auch die Lösung des Silbersalzes zum Fällungsreagenz. Weiterhin kann das Fällungsreagenz in fester oder flüssiger Form verdünnt oder unverdünnt eingesetzt werden.

Als bevorzugte Ausfällreagenzien können verwendet werden Oxalsäure oder deren Salze, die besonders bevorzugt ist, gesättigte oder ungesättigte Mono-Carbonsäuren wie Ameisensäure oder deren Salze, Essigsäure oder deren Salze, Propionsäure oder deren Salze, Buttersäure oder deren Salze oder Milchsäure oder deren Salze.

Ebenfalls geeignet sind Benzoesäure und Benzoeate und Salicylsäure und deren Salze. Des weiteren sind hier zu nennen gesättigte oder ungesättigte Dicarbonsäuren wie beispielsweise Fumarsäure und deren Salze (Fumarate) und Maleinsäure und deren Salze (Maleinate). Schließlich seien noch gesättigte oder ungesättigte Tri-Carbonsäuren, wie z. B. Zitronensäure oder deren Salze genannt.

Nach der Ausfällung der schwerlöslichen Silberverbindung nach der beschriebenen Verfahrensweise können die schwerlöslichen Silberverbindungen durch Abfiltrieren, Absaugen über Filternutschen oder andere geeignete Verfahren aus der Lösung isoliert und unmittelbar anschließend in Kontakt mit dem Komplexbildner gebracht werden. Diese Verfahrensweise hat bei schwerlöslichen Silberverbindungen die eine gewisse Instabilität aufweisen oder in der Handhabung Risiken in sich bergen, Vorteile. Bei der bevorzugten Verwendung von Silberoxalat als schwerlösliche Silberverbindung in der erfindungsgemäßen Verwendung wird die vorherige in situ-Herstellung des Oxalats besonders bevorzugt.

Besonders vorteilhaft können erfindungsgemäß die nach der vorstehenden Verfahrensweise erhältlichen geträgerten silberhaltigen Katalysatoren für die Herstellung von 3-Methylbut-2-en-1-al aus 3-Methylbut-3-en-1-ol eingesetzt werden. Das Produkt ist auch unter dem Trivialnamen Prenal, das Edukt unter dem Trivialnamen Isoprenol, bekannt.

Bei dieser besonders bevorzugten Verwendung wird die Umsetzung vorzugsweise in einem Rohrbündelreaktor, wie er z. B. in der EP-A 881 206 beschrieben ist, durchgeführt. Wegen weiterer Einzelheiten zur Reaktorgeometrie sei hier auf diese EP-A 881 206 und die EP-A 244 632 verwiesen.

Durch die erfindungsgemäße Verwendung der wie vorstehend beschrieben erhältlichen silberhaltigen geträgerten Katalysatoren ist es möglich, Prenal aus Isoprenol unter milden Temperaturbedingungen mit guter Ausbeute und guter Selektivität zu erhalten. Bei der Umsetzung von Isoprenol mit dem wie vorstehend beschrieben erhältlichen silberhaltigen geträgerten Katalysator entsteht ein Reaktionsgemisch aus 3-Methylbut-3-en-1-al und 3-Methylbut-2-en-1-al. Das erstgenannte Isomere isomerisiert dann basenkatalysiert in einem Folgeschritt zum gewünschten 3-Methylbut-2-en-1-al.

Bei der Aufarbeitung des Reaktionsgemisches wird in einer ersten Stufe das gewünschte Reaktionsprodukt von nicht umgesetztem Edukt destillativ getrennt. Um diese Destillation wirtschaftlich vorteilhaft durchführen zu können, macht man sich vorteilhaft ein Azeotrop zu Nutze, welches aus 70% 3-Methylbut-3-en-1-al und 30% 3-Methylbut-2-en-1-al besteht. Letzteres ist, wie vorstehend erwähnt, das thermodynamisch begünstigte Produkt.

Nach der erfindungsgemäßen Verwendung des wie vorstehend beschrieben erhältlichen geträgerten silberhaltigen Katalysators kann Prenal in guter Ausbeute bei niedrigeren Temperaturen und mit guter Selektivität aus Isoprenol hergestellt werden.

Die nachfolgenden Beispiele verdeutlichen die Vorteile der erfindungsgemäßen Verwendung.

### Beispiel 1: (Stand der Technik)

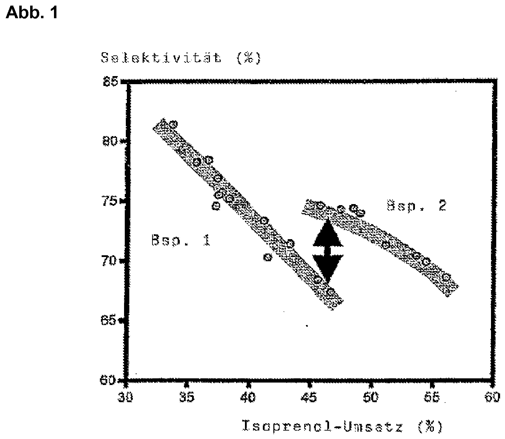

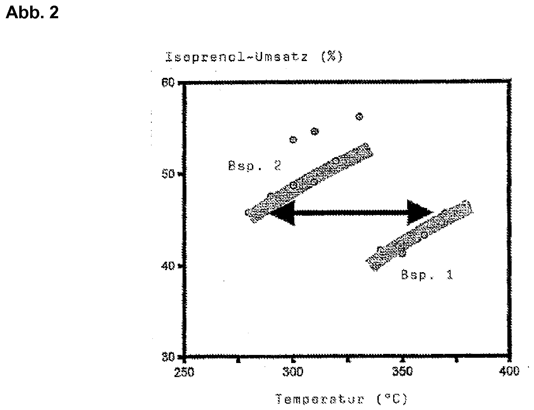

Steatit-Kugeln mit einem Durchmesser von 1,8 bis 2,2 mm (Hersteller Firma Ceram Tec) wurden mit Silber nach dem sog. Flammenspritzverfahren über eine Acetylen-Flamme (wie beispielhaft in Army Engineering Manual EM 1110-2-3401 beschrieben) beschichtet. Die Silber-Beladung des Katalysators nach der Beschichtung betrug 6 Gew.-%. Der so erhaltene Katalysator wurde in einer Versuchsanlage aus einem über ein Sandbad beheizten Quarzglas-Rohrreaktor mit 13 mm Innendurchmesser und 150 mm Länge getestet. Dazu wurde der Reaktor mit einer Schütthöhe des Katalysators von 100 mm befüllt. Über dieses Katalysatorbett wurden pro Stunde 110 g verdampftes Isoprenol und 50 NI [NI = Normliter, das Volumen von einem Liter unter Standardbedingungen] Luft als Gas geleitet. Abb. 1 zeigt die Selektivität als Funktion des Isoprenol-Umsatzes und Abb. 2 den Isoprenol-Umsatz als Funktion der Temperatur.

### Beispiel 2:

Steatit-Kugeln mit einem Durchmesser von 1,5 bis 1,8 mm (Hersteller Firma Ceram Tec) wurden durch Aufbringen einer mit Ethylendiamin komplexierten Lösung von Silberoxalat benetzt. Die so mit der Silberlösung benetzten Steatit-Kugeln wurden anschließend im Luftstrom bei 280°C für 12 Minuten behandelt. Der spezifische Widerstand des so erhaltenen Katalysators lag bei 5 mΩ ^{∗}m.

Der so erhaltene Katalysator wurde in der Versuchsanlage aus Beispiel 1 auf seine katalytische Wirkung hin untersucht. Dabei wurde die gleiche Schütthöhe und der gleiche Durchsatz an Isoprenol und Luft wie in Beispiel 1 verwendet.

In den Abbildungen 1 und 2 sind die Selektivität als Funktion des Isoprenol-Umsatzes und der Isoprenol-Umsatz als Funktion der Temperatur der Umsetzungen gemäß Beispiel 1 und Beispiel 2 gegenüberstellt. Wie aus den Abbildungen 1 und 2 eindeutig hervorgeht, wird nach Beispiel 2 eine verbesserte katalytische Wirksamkeit hinsichtlich Selektivität und Aktivität gegenüber Katalysator nach Beispiel 1 erreicht.

## Patentansprüche

1. Verwendung eines geträgerten silberhaltigen Katalysators, erhältlich durch
a) Aufbringen einer komplexierten schwerlöslichen Silberverbindung aus Suspension oder Lösung auf Steatit als Trägermaterial,
b) anschließende thermische Behandlung des nach Stufe a) erhaltenen Produkts bei Temperaturen im Bereich von 100 bis 400°C über einen Zeitraum von 5 min bis 5 h,
wodurch aus der Silberverbindung durch Reduktion das elementare Edelmetall entsteht,
zur Herstellung von olefinisch ungesättigten Carbonylverbindungen aus olefinisch ungesättigten Alkoholen durch oxidative Dehydrierung,
wobei die Komplexierungsmittel amin-, hydroxy- und carboxylgruppenhaltige Kohlenwasserstoffe, Ammoniak oder Oxo-, Aza- oder Thio-Makrozyklen sind, und wobei
als schwerlösliche Silberverbindungen solche mit einer Löslichkeit in wässriger Lösung bei einer Temperatur von 25°C und einem pH-Wert von 7 von weniger als 5,0 g/l eingesetzt werden.

2. Verwendung nach Anspruch 1, **dadurch gekennzeichnet, dass** die Silberverbindung in Mischung mit als Promotoren wirkenden Zusätzen aufgebracht wird.

3. Verwendung nach Anspruch 1 oder 2, **dadurch gekennzeichnet, dass** 3-Methylbut-2-en-1-al aus 3-Methylbut-3-en-1-ol hergestellt wird.

4. Verfahren zur Herstellung eines geträgerten silberhaltigen Katalysators zur oxidativen Dehydrierung von olefinisch ungesättigten Alkoholen, **dadurch gekennzeichnet, dass**
a) eine komplexierte schwerlösliche Silber-Verbindung aus Suspension oder Lösung auf Steatit als Trägermaterial aufgebracht wird, und anschließend
b) das in Schritt a) erhaltene Produkt bei Temperaturen im Bereich von 100 bis 400°C über einen Zeitraum von 5 min bis 5 h thermisch behandelt wird,
wodurch aus der Silberverbindung durch Reduktion das elementare Edelmetall entsteht,
wobei die Komplexierungsmittel amin-, hydroxy- und carboxylgruppenhaltige Kohlenwasserstoffe, Ammoniak oder Oxo-, Aza- oder Thio-Makrozyklen sind, und wobei
als schwerlösliche Silberverbindungen solche mit einer Löslichkeit in wässriger Lösung bei einer Temperatur von 25°C und einem pH-Wert von 7 von weniger als 5,0 g/l eingesetzt werden.

5. Verfahren nach Anspruch 5, **dadurch gekennzeichnet, dass** die Silberverbindung in Mischung mit als Promotoren wirkenden Zusätzen aufgebracht wird.

6. Geträgerter silberhaltiger Katalysator mit einem spezifischen Widerstand von nicht mehr als 1000 mΩ^{∗}m, erhältlich durch
a) Aufbringen einer komplexierten schwerlöslichen Silber-Verbindung aus Suspension oder Lösung auf Steatit als Trägermaterial,
b) anschließende thermische Behandlung des nach Stufe a) erhaltenen Produkts bei Temperaturen im Bereich von 100 bis 400°C über einen Zeitraum von 5 min bis 5 h,
wodurch aus der Silberverbindung durch Reduktion das elementare Edelmetall entsteht,
wobei die Komplexierungsmittel amin-, hydroxy- und carboxylgruppenhaltige Kohlenwasserstoffe, Ammoniak oder Oxo-, Aza- oder Thio-Makrozyklen sind, und wobei
als schwerlösliche Silberverbindungen solche mit einer Löslichkeit in wässriger Lösung bei einer Temperatur von 25°C und einem pH-Wert von 7 von weniger als 5,0 g/l eingesetzt werden.

7. Geträgerter silberhaltiger Katalysator nach Anspruch 6, **dadurch gekennzeichnet, dass** die Silberverbindung in Mischung mit als Promotoren wirkenden Zusätzen aufgebracht wird.

## Claims

1. The use of a supported silver catalyst, obtainable by
a) applying a complexed sparingly soluble silver compound to steatite as support material from suspension or solution,
b) subsequently thermally treating the product obtained in stage a) at temperatures in the range from 100 to 400°C over a period of 5 min to 5 h,
which forms the elemental noble metal from the silver compound by reduction,
for preparing olefinically unsaturated carbonyl compounds from olefinically unsaturated alcohols by oxidative dehydrogenation,
the complexing agents being amine-, hydroxyl- and carboxyl-containing hydrocarbons, ammonia, or oxo, aza or thio macrocycles, and
the sparingly soluble silver compounds used being those having a solubility in aqueous solution at a temperature of 25°C and a pH of 7 of less than 5.0 g/l.

2. The use according to claim 1, wherein the silver compound is applied in a mixture with additives which act as promoters.

3. The use according to claim 1 or 2, wherein 3-methylbut-2-en-1-al is prepared from 3-methylbut-3-en-1-ol.

4. A process for producing a supported silver catalyst for oxidative dehydrogenation of olefinically unsaturated alcohols, which comprises
a) applying a complexed sparingly soluble silver compound to steatite as support material from suspension or solution, and then
b) thermally treating the product obtained in step a) at temperatures in the range from 100 to 400°C over a period of 5 min to 5 h,
which forms the elemental noble metal from the silver compound by reduction,
the complexing agents being amine-, hydroxyl- and carboxyl-containing hydrocarbons, ammonia, or oxo, aza or thio microcycles, and
the sparingly soluble silver compounds used being those having a solubility in aqueous solution at a temperature of 25°C and a pH of 7 of less than 5.0 g/l.

5. The process according to claim 4, wherein the silver compound is applied in a mixture with additives which act as promoters.

6. A supported silver catalyst having a specific resistivity of not more than 1000 mΩ^{∗}m, obtainable by
a) applying a complexed sparingly soluble silver compound to steatite as support material from suspension or solution,
b) subsequently thermally treating the product obtained in step a) at temperatures in the range from 100 to 400°C over a period of 5 min to 5 h,
which forms the elemental noble metal from the silver compound by reduction,
the complexing agents being amine-, hydroxyl- and carboxyl-containing hydrocarbons, ammonia, or oxo, aza or thio microcycles, and
the sparingly soluble silver compounds used being those having a solubility in aqueous solution at a temperature of 25°C and a pH of 7 of less than 5.0 g/l.

7. The supported silver catalyst according to claim 6, wherein the silver compound is applied in a mixture with additives which act as promoters.

## Revendications

1. Utilisation d'un catalyseur supporté, contenant de l'argent, pouvant être obtenu par
a) application d'un composé à base d'argent, complexé, peu soluble, à partir d'une suspension ou d'une solution sur de la stéatite comme matériau support,
b) traitement thermique consécutif du produit obtenu après l'étape a) à des températures dans la plage de 100 à 400°C sur une période de 5 min à 5 h,
le métal noble élémentaire étant obtenu par réduction à partir du composé à base d'argent,
pour la préparation de composés carbonyle oléfiniquement insaturés à partir d'alcools oléfiniquement insaturés par déshydrogénation oxydante,
les complexants étant des hydrocarbures contenant des groupes amine, hydroxy et carboxyle, l'ammoniaque ou des macrocycles à fonction oxo, aza ou thio et
des composés d'argent présentant une solubilité en solution aqueuse, à une température de 25°C et à un pH de 7, inférieure à 5,0 g/l étant utilisés comme composés à base d'argent peu solubles.

2. Utilisation selon la revendication 1, **caractérisée en ce que** le composé à base d'argent est appliqué en mélange avec des additifs agissant comme promoteurs.

3. Utilisation selon la revendication 1 ou 2, **caractérisée en ce que** du 3-méthylbut-2-én-1-al est préparé à partir de 3-méthylbut-3-én-1-ol.

4. Procédé pour la préparation d'un catalyseur contenant de l'argent, supporté, pour la déshydrogénation oxydante d'alcools oléfiniquement insaturés, **caractérisé en ce que**
a) un composé à base d'argent, complexé, peu soluble, est appliqué à partir d'une suspension ou d'une solution sur de la stéatite comme matériau support et ensuite
b) le produit obtenu dans l'étape a) est traité thermiquement à des températures dans la plage de 100 à 400°C sur une période de 5 min à 5 h,
le métal noble élémentaire étant obtenu par réduction à partir du composé à base d'argent,
les complexants étant des hydrocarbures contenant des groupes amine, hydroxy et carboxyle, l'ammoniaque ou des macrocycles à fonction oxo, aza ou thio et
des composés d'argent présentant une solubilité en solution aqueuse, à une température de 25°C et à un pH de 7, inférieure à 5,0 g/l étant utilisés comme composés à base d'argent peu solubles.

5. Procédé selon la revendication 4, **caractérisé en ce que** le composé à base d'argent est appliqué en mélange avec des additifs agissant comme promoteurs.

6. Catalyseur supporté contenant de l'argent, présentant une résistance spécifique qui n'est pas supérieure à 1000 mΩ^{∗}m, pouvant être obtenu par
a) application d'un composé à base d'argent, complexé, peu soluble, à partir d'une suspension ou d'une solution sur de la stéatite comme matériau support,
b) traitement thermique consécutif du produit obtenu après l'étape a) à des températures dans la plage de 100 à 400°C sur une période de 5 min à 5 h,
le métal noble élémentaire étant obtenu par réduction à partir du composé à base d'argent,
les complexants étant des hydrocarbures contenant des groupes amine, hydroxy et carboxyle, l'ammoniaque ou des macrocycles à fonction oxo, aza ou thio et
des composés d'argent présentant une solubilité en solution aqueuse, à une température de 25°C et à un pH de 7, inférieure à 5,0 g/l étant utilisés comme composés à base d'argent peu solubles.

7. Catalyseur supporté contenant de l'argent selon la revendication 6, **caractérisé en ce que** le composé à base d'argent est appliqué en mélange avec des additifs agissant comme promoteurs.
